# EUROPEAN PATENT APPLICATION

(11) **EP 4 582 082 A1**
(43) Date of publication of application: **09.07.2025**
(21) Application number: 23859998.9
(22) Date of filing: 09.08.2023
(51) Int. Cl.: A61K 9/14, A61K 45/00, A61K 47/20, A61K 47/22, A61K 49/00, A61P 29/00

(54) **PAIN THERAPEUTIC AGENT, AND AGENT FOR DETERMINING ADMINISTRATION SITE OF PARTICULATE EMBOLIC MATERIAL**

(30) Priority: 31.08.2022 JP 2022138457
(71) Applicant: Medical Corporation Yuyukai, Tokyo 106-0032 (JP)
(72) Inventor: OKUNO, Yuji, Tokyo 106-0032 (JP)
(74) Representative: HGF
(86) International application number: PCT/JP2023/029123
(87) International publication number: WO 2024/048232

(57) **Abstract**

The present invention addresses the problem of providing a technique for treating a sports injury or a pain associated with a sports injury. Provided is a therapeutic agent for a sports injury or a pain associated with a sports injury, the therapeutic agent containing a particulate embolic material having an average diameter of 10-500 um inclusive, and the therapeutic agent being administered into an artery that nourishes the affected area of the sports injury and/or into an artery that nourishes the painful area associated with the sports injury.

## Description

### TECHNICAL FIELD

The present invention relates to a pain therapeutic agent and an agent for determining administration site of particulate embolic material.

### BACKGROUND ART

Although the causes of pains are diverse, the present inventor has found that one cause is abnormal blood flow due to an increase in abnormal capillary vessels (known as "problematic vessels".) (e.g., Non-Patent Document 1). Such abnormal capillary vessels are generated by nerve growth and the like.

Based on the above finding, the present inventor established a method for treating a pain by delivering an embolic material (e.g., imipenem/cilastatin particles) to abnormal capillary vessels to embolize the abnormal capillary vessels.

### Citation List

### Non-Patent Document

Non-Patent Document 1: Yuji Okuno; "HEBAHDEN KESSETSU NO ITAMIWA MOYAMOYAKEKKAN GA GENIN DATTA (PAIN OF HEBERDEN'S NODULE WAS CAUSED BY PROBLEMATIC VESSELS)" 2020, WANI BOOKS

### DISCLOSURE OF THE INVENTION

### Problems to be Solved by the Invention

Incidentally, the present inventor has found that abnormal capillary vessels are also formed in patients having a so-called "sports injury" caused by excessive exercise.

However, in sports injuries, it has been found that accurate identification of abnormal capillary vessels to be embolized is difficult, and there is a concern that administration of an excessive amount of embolic material to an inappropriate site ends up worsening the state of disease.

The present invention has been made in view of the above circumstance, and an object of the present invention is to provide a technique for treating a sports injury or a pain due to the sports injury.

### Means for Solving the Problems

The present inventor has developed a therapeutic agent suitable for the treatment of sports injuries, and has found a method for determining an appropriate administration site of the therapeutic agent, thereby arriving at completion of the present invention. More specifically, the present invention provides the following.

<1> A therapeutic agent for a sports injury or a pain due to the sports injury,
   the therapeutic agent including a particulate embolic material having an average particle size of 10 µm or more and 500 µm or less,
   in which the therapeutic agent is administered in an artery that feeds an affected area of the sports injury and/or in an artery that feeds a pain site due to the sports injury.
<2> A therapeutic agent for a sports injury or a pain due to the sports injury,
   including a particulate embolic material having an average particle size of 10 µm or more and 500 µm or less,
   in which the therapeutic agent is administered to one or more of the following sites:
      (1) a site for which an abnormal finding is recognized based on imaging of blood vessels at a candidate administration site located in an artery that feeds an affected area of the sports injury and/or in an artery that feeds a pain site due to the sports injury or
      (2) a site where a sense of discomfort occurs in an affected area after a contrast agent with a higher osmotic pressure than blood or an embolic material with a higher osmotic pressure than blood is administered to the candidate administration site.
<3> A particulate embolic material administration site determination agent for treating a sports injury or a pain due to the sports injury,
   the agent including a contrast agent with a higher osmotic pressure than blood or an embolic material with a higher osmotic pressure than blood,
   in which the agent is used for determining a candidate administration site of the particulate embolic material based on whether or not the agent administered to the candidate administration site causes a sense of discomfort in an affected area,
   the candidate administration site is located in an artery that feeds the affected area of the sports injury and/or in an artery that feeds a pain site due to the sports injury, and the particulate embolic material has an average particle diameter of 10 µm or more and 500 µm or less.

### Effects of the Invention

According to the present invention, techniques are provided for treating a sports injury or a pain due to the sports injury.

### PREFERRED MODE FOR CARRYING OUT THE INVENTION

Hereinafter, embodiments of the present invention will be described, but the present invention is not limited thereto.

### <Therapeutic Agents>

The therapeutic agent of the present invention is an agent for treating a sports injury or a pain due to the sports injury. The therapeutic agent includes a particulate embolic material having an average particle size of 10 µm or more and 500 µm or less, and is administered into an artery that feeds an affected area of a sports injury and/or into an artery that feeds a pain site due to the sports injury.

As described above, the present inventor developed a method of treating a pain, the method including injecting an embolic material into an abnormal capillary vessel to embolize the abnormal capillary vessel to normalize the blood flow. Then, the present inventor discovered that the etiology of a sports injury is abnormal capillary growth, and has found a novel finding that the above-described treatment method can treat a sports injury or a pain due to the sports injury.

### (1) Sports injuries

In the present invention, the term "sports injury" encompasses disorders caused by various exercises (ball game, gymnastics, etc.) with a large load on the body. The presence or absence or type of sports injury can be identified by diagnosis by a physician.

In the present invention, the term "sports injury" includes both those accompanied by a pain and those not accompanied by a pain. When a sports injury is accompanied by pain, the pain is also the subject of the therapy of the present invention.

The affected area of a sports injury encompasses any site. For example, the affected area of a sports injury may be a shoulder, an elbow, a lower back, an upper back, a hip joint, a knee joint, a foot joint, a foot, or a hand joint.

Where the affected area of the sports injury is the shoulder, the sports injury may include a disabled throwing shoulder, a shoulder impingement syndrome, a painful rotator cuff tear, a SLAP lesion, rotator interval inflammation, biceps long head tendinitis, calcific tendinitis, and rotator cuff tendinitis.

Where the affected area of the sports injury is the elbow, the sports injury includes a medial ulnar collateral ligament injury, olecranon stress fracture, lateral humeral epicondylitis, medial humeral epicondylitis, an infrapatellar synovial fold injury, elbow-joint synovial inflammation, osteochondritis dissecans, and other pains in the elbow joint due to sports.

Where the affected area of the sports injury is the lower back and/or the upper back, the sports injury includes lumbar spondylolysis, intervertebral joint backache, lumbar disc arthritis, thoracic disc arthritis, fascia of muscles backache, interspinal desmitis, lumber disc herniation, lumbar disc endplate inflammation, sacroiliac joint inflammation, and other backaches due to sports.

When the affected part of the sports injury is the hip joint, the sports injury includes hamstrings enthesitis, hip joint synovitis, a hip joint lip lesion, a hip joint impingement syndrome, a groin pain syndrome, great trochanter bursitis, and other pains of the hip joint due to sports.

When the affected part of the sports injury is a knee joint, the sports injury includes patellar tendinitis, patellofemoral desmitis, Iliotibial desmitis, pes anserinus bursitis, subpatellar panniculitis, a medial collateral ligament lesion, a semi-lunar disc lesion, Osgood disease, a synovial membrane injury, patella partita, and other pains in the knee joint due to sports.

Where the affected area of the sports injury is a foot joint and/or foot, the sports injury may include Achilles tendinitis, plantar aponeurosis, painful os tibiale externum, foot joint impingement syndrome, Hallux valgus, Morton's neuroma, tibialis posterior tendinitis, foot joint synovitis, MP arthritis, and other pains in the foot joint or foot due to sports.

Where the affected area of the sports injury is a hand joint, the sports injury may include a TFCC lesion, tendon sheath inflammation, navicular bone fracture, hand joint synovitis, and other pains in the hand joint due to sports.

Other sports injuries include a repetitive muscular detachment, fatigue fracture, fracture healing delay, a false joint, and the like.

### (Embolic Material)

As the embolic material, a particulate material having an average particle size of 10 µm or more and 500 µm or less is used.

The phrase "embolic material" as used herein means a material capable of blocking a blood flow in an artery.

An average particle diameter of the embolic material can be appropriately adjusted depending on a diameter, etc. of the artery to be embolized.

From the viewpoint that the artery to be embolized is easily embolized, a lower limit of the average particle diameter of the embolic material is preferably 30 µm or more and more preferably 50 µm or more.

From the viewpoint of easiness in avoiding embolizing an unintended artery other than the abnormal capillary vessel or of easiness of delivery to the artery to be embolized, an upper limit of the average particle diameter of the embolic material is preferably 350 µm or less and more preferably 250 µm or less.

The "average particle diameter" as used herein indicates an average value of particle size distribution and is determined using a laser diffraction-type particle size distribution measuring device (e.g., "SALD" series, manufactured by SHIMADZU CORPORATION).

The shape of the embolic material is not particularly limited, but may be amorphous, spherical, rectangular, etc.

The embolic material is not particularly limited as long as it can block a blood flow in an artery and does not adversely affect a living body.

A material of the embolic material may be a material sparingly soluble in blood at a body temperature (e.g., 35 to 39°C), in particular, imipenem/cilastatin, etc. However, from the viewpoint of preventing a normal blood vessel from being embolized when the embolic material unintentionally enters a normal blood vessel, the embolic material may also be a material temporarily sparingly soluble in blood at a body temperature (e.g., 35 to 39°C) but eventually soluble in the blood at the body temperature.

One preferred aspect of the present invention includes an aspect in which the embolic material is a particulate embolic material containing a water-soluble polymer and satisfying both of the following (A) and (B):
(A) 0.2 g of the material is mixed in 30 mL of physiological saline at 37°C, and the "diffraction intensity/scattered light intensity" after 10 minutes is 10% or more of the "diffraction intensity/scattered light intensity" immediately after mixing, and
(B) 0.2 g of the material is mixed in 30 mL of physiological saline at 37°C, and the "diffraction intensity/scattered light intensity" after one hour is 90% or less of the "diffraction intensity/scattered light intensity" immediately after mixing. (The "diffraction intensity/scattered light intensity" is measured using a laser diffraction particle size distribution analyzer "SALD-2300" (manufactured by Shimadzu Corporation) and a batch cell "BC23" (manufactured by Shimadzu Corporation) at a refractive index of 1.65 to 0.20i.)

The "diffraction intensity/scattered light intensity" is a value obtained by dividing the diffraction intensity of the particulate embolic material by the scattered light intensity of the particulate embolic material. In the particulate embolic material satisfying both (A) and (B), the "diffraction intensity/scattered light intensity" at three time points **(i.e.,** immediately after preparation, 10 minutes after preparation, and 1 hour after preparation) in the preparation of the physiological saline solution are used as indexes of an appropriate embolic material size adjusted to the progress after the administration.

In the comparison of "diffraction intensity/scattered light intensity" at each time point, first of all, among the sensors of "SALD-2300" (having 65 sensors), a sensor that shows the highest "diffraction intensity/scattered light intensity" in the measurement immediately after mixing is identified. Next, with regard to the sensor, comparison is made between "diffraction intensity/scattered light intensity" immediately after mixing and "diffraction intensity/scattered light intensity" at a subsequent time point (after 10 minutes or after 1 hour).

When (A) is less than 10%, the particulate embolic material dissolves too quickly in the body, and the embolic material does not easily embolize abnormal blood vessels, providing a less effective pain therapeutic result. Therefore, (A) is preferably 30% or more, and more preferably 50% or more. The upper limit of (A) is not particularly limited, but is preferably 100% or less, more preferably 98% or less, and even more preferably 95% or less from the viewpoint of easiness in satisfying the appropriate embolic material size. However, (A) has a higher value than (B) described later.

When (B) is more than 90%, the particulate embolic material does not easily dissolve in the body, and easily embolizes normal blood vessels that feed joints, so that strong side effects such as a pain after administration are likely to occur. Therefore, (B) is preferably 70% or less, and more preferably 30% or less. The lower limit of (B) is not particularly limited, but is preferably 1% or more, more preferably 3% or more, and even more preferably 5% or more from the viewpoint of easiness in satisfying the appropriate embolic material size.

A ratio between (A) and (B) is not particularly limited as long as (A)>(B) is satisfied. The lower limit of "(A)/(B)" (a value obtained by dividing (A) by (B)) may be, for example, 1.01 or more, 1.50 or more, 2.00 or more, or 3.00 or more. The upper limit of "(A)/(B)" may be, for example, 10.00 or less, 9.00 or less, 8.00 or less, 7.00 or less, 6.00 or less, or 5.00 or less.

The water-soluble polymer is not particularly limited as long as it has gelling ability, and examples thereof include non-crosslinked polymers such as agar, gelatin, collagen, and curdlan, and crosslinked polymers such as sodium alginate, sodium polyacrylate, pectin, and carrageenan. In addition, the embolic material may or may not include a polymer other than the water-soluble polymer. Such a water-soluble polymer is dispersed in water to form a three-dimensional structure, and a hydrogel of a short-time dissolution type may be used as an embolic material in one embodiment. Although not particularly limited, the embolic material satisfying the conditions (A) and (B) can be produced by appropriately adjusting the hydrophilicity, molecular weight, and crosslinking density of the water-soluble polymer. Commercially available products of such embolic materials include "IPZA100-300" and "IPZA300-500" manufactured by Engain, and "Nexsphere" manufactured by Nextbiomedical.

The therapeutic agent of the present invention is an agent to be used by injection administration to an artery. Therefore, the therapeutic agent of the present invention may include, in addition to the embolic material, a medium that can be administered to an artery of a living body (physiological buffer, sterile water, saline, a medium, a contrast agent) and an optional component that may be included in injection agents if needed as long as the embolizing effect of the embolic material is not impaired.

The form of the therapeutic agent of the present invention may be an injection agent. The configuration of the injection is not particularly limited, but any configuration may be adopted that allows the therapeutic agent to be administered to a target of the administration, using an injection needle. Normally, the injection includes an injection needle (Surflo indwelling needle, or the like), a syringe or the like, and the embolic material or the like is filled in the syringe.

When the therapeutic agent of the present invention is administered, angiography may be performed in order to identify an administration site of the therapeutic agent. Therefore, a contrast agent may be used in combination with the embolic material of the present invention for the angiography. The type of the contrast agent is not particularly limited, but iohexol or the like may be used. It should be noted that the contrast agent may be included in the therapeutic agent of the present invention (that is, the contrast agent may be administered simultaneously with the embolic material) or may be administered separately from the therapeutic agent of the present invention. When the contrast agent is administered separately from the therapeutic agent of the present invention, the order of administration is not particularly limited, but preferably the contrast agent is administered first from the viewpoint of easiness of identification of the administration site.

### (Administration Site)

The therapeutic agent of the present invention is administered into an artery that feeds an affected area of the sports injury and/or an artery that feeds a pain site due to the sports injury.

Individual arteries listed below include not only the artery but also branches branching from the artery.

From the viewpoint that the embolic material can be easily administered to the abnormal capillary vessels (problematic vessel), the administration site may be an artery that feeds the pain site. The "artery that feeds the pain site" may be specifically an artery that is distributed within 10 cm upstream from the pain site.

In intra-arterial administration, the therapeutic agent of the invention may be administered using a catheter.

The diameter, shape and the like of the catheter are not particularly limited as long as it is a tube having a luminal structure capable of being inserted into an artery and capable of injecting an embolic material. In general, a catheter (so-called microcatheter) having an outer diameter of 0.6 to 1 mm can be suitably used.

In the operation of the catheter, a device or a method generally employed in transcatheter administration, such as a guide wire or a pump, can be used.

During administration from a catheter, press-injection or free flow can be appropriately selected according to the site of administration or the like. For example, administration by press-injection is preferred where there is communication between arteries. In this case, it is desirable to confirm that the distance (e.g., 2 cm or more) from the spinal artery is ensured. For example, free flow is preferred from the viewpoint of reducing the risk of the embolic material flowing into the spinal artery due to backflow by press-injection.

In the present invention, as a method of avoiding backflow during administration from the catheter, there is a method of administering an embolic material to a site where it has been confirmed that a test injected contrast agent naturally flows into peripheral blood vessels (also called washout).

In the injection administration, the configuration of the injection agent is not particularly limited, but any configuration that is suitable for arterial injection therapy (puncturing an artery directly with a needle without using a catheter or the like) and can administer the therapeutic agent to an administration subject using an injection needle can be adopted. Normally, an injection agent includes an injection needle (such as a Surflo indwelling needle), a syringe, and the like, and the syringe is filled with an embolic material or the like.

When the affected area of a sports injury is the shoulder, an artery that feeds the affected area of the sports injury and/or an artery that feeds the pain site due to the sports injury include a subscapular artery, an acromiothoracic artery, a coracoid branch, a scapular circumflex artery, an anterior circumflex humeral artery, and a posterior circumflex humeral artery.

When the affected area of the sports injury is the elbow, the artery that feeds the affected area of the sports injury and/or the artery that feeds the pain site due to the sports injury include an arteria profunda brachii, a radial collateral artery, an ulnar collateral artery, a radial recurrent artery, a recurrent interosseous artery, and a recurrent ulnar artery.

When the affected area of a sports injury is the lower back and/or the upper back, the arteries that feed the affected areas of the sports injury and/or the arteries that feed the pain site due to the sports injury include left and right first to fourth lumbar arteries, an iliolumbar artery, a middle sacral artery, a lateral sacral artery, a superior gluteal artery, an inferior gluteal artery, and an intercostal artery.

When the affected area of the sports injury is the hip joint, the arteries that feed the affected area of the sports injury and/or the arteries that feed the pain site due to the sports injury include a superior gluteal artery, an inferior gluteal artery, an obturator artery, a deep iliac circumflex artery, an inferior epigastric artery, a medial circumflex femoral artery, and a lateral circumflex femoral artery.

When the affected part of the sports injury is the knee joint, the arteries that feed the affected area of the sports injury and/or the arteries that feed the pain site due to the sports injury include a descending artery of knee, an upper medial artery of knee, a superolateral artery of knee, a median artery of knee, a lower lateral artery of knee, an inferior medial artery of knee, an anterior tibial recurrent artery, and a suprapatellar artery.

When the affected area of the sports injury is the foot joint and/or the foot, the arteries that feed the affected area of the sports injury and/or the arteries that feed the pain site due to the sports injury include an anterior tibial artery, a posterior tibial artery, and a peroneal artery.

When the affected part of the sports injury is the hand joint, the arteries that feed the affected area of the sports injury and/or the arteries that feed the pain site due to the sports injury include a radial artery, an ulnar artery, and an interosseous artery.

When the sports injury is repetitive muscle detachment, fatigue fracture, fracture healing delay, or a pseudo-joint, since the arteries that feed the affected area of the sports injury and/or the arteries that feed the pain site due to the sports injury vary depending on the site, the artery (candidate administration site) considered to feed the affected area is selected, the catheter is delivered thereto, angiography is selectively performed and the administration site is identified.

### (4) Identification of Administration Site

A sports injury or a pain due to the sports injury may be difficult to identify its responsible vessels. If the therapeutic agent of the present invention is administered to a site other than the responsible blood vessels, the blood vessels that are inappropriate for therapy may be embolized, or an excessive amount of the therapeutic agent may be administered, which may exacerbate the injury and the pain.

Therefore, as a result of study by the present inventor, it has been found that the above-described chance can be avoided by administering the therapeutic agent to one or more of the following sites:
site 1: a site for which an abnormal finding is recognized based on imaging of blood vessels at a candidate administration site located in the arteries that feed an affected area of a sports injury and/or in arteries that feed a pain site due to the sports injury or
site 2: a site where a sense of discomfort occurs in the affected area after a contrast agent with a higher osmotic pressure than blood or an embolic material with a higher osmotic pressure than blood is administered to the candidate administration site in the site 1.

In the identification of any of the above sites, a "candidate administration site" for administration is initially selected, the "candidate administration site" being located in arteries that feed the affected area of the sports injury and/or in arteries that feed the pain site due to the sports injury. It is determined in the following manner whether this candidate administration site is actually responsible vessels for the sports injury or the pain due to the sports injury. As a result of the determination, when it does not correspond to either the site 1 or the site 2, the therapeutic agent is not administered, another candidate administration site is selected, and a search for responsible blood vessels is performed based on whether it corresponds to the site 1 or the site 2.

### (4-1) Regarding Site 1

For the candidate administration site, the vessels are imaged by an arbitrary means. For example, the imaging may employ the following steps. First, a catheter is delivered to the candidate administration site, and an arbitrary contrast agent is released to the candidate administration site. X-ray imaging of the candidate administration site is then performed to obtain an X-ray image. Based on the obtained image, the presence or absence of abnormal finding at the candidate administration site is confirmed. Examples of the abnormal finding include the following.
- Deep staining image: an observation where as a result of an increase in abnormal blood vessels, the contrast agent is distributed more in the affected area than in the normal area, making it appear as if only that area is stained.
- Early venous filling image: a phenomenon in which arteries and veins are abnormally connected to each other and, as a result, the veins are seen at a timing earlier than a normal timing at which the veins are seen after the contrast agent slowly passes through the capillary blood vessels from the arteries (for example, during artery imaging). If an abnormal finding is found based on the image, the candidate administration site can be determined as the responsible blood vessel. Therefore, the site is identified as the administration site (the above-mentioned site 1), and the therapeutic agent of the present invention and the particulate embolic material administration site determination agent described later are administered while the catheter is disposed at the candidate administration site as it is. If based on the image, it is clear that there are no abnormal findings, the catheter is removed from the candidate administration site, delivered to another candidate administration site, and the above procedure is performed again.

### (4-2) Regarding Site 2

Even if the presence or absence of abnormal findings at the candidate administration site is unclear based on imaging, there is still a chance that the candidate administration site is an effective administration site. In such a case, a contrast agent with a higher osmotic pressure than blood or an embolic material with a higher osmotic pressure than blood is administered to the candidate administration site, to confirm whether or not a sense of discomfort occurs in the affected area.

Examples of the sense of discomfort caused by administration of the contrast agent with a higher osmotic pressure than blood or an embolic material with a higher osmotic pressure than blood include a pain (reproduced pain) occurring at the same place as a place of the pain due to the sports injury, a heat sensation occurring at the same place as a place of the pain due to the sports injury, itching occurring at the same place as a place of the pain due to the sports injury, and the like. In addition, the timing at which the sense of discomfort occurs may be, for example, any time from immediately after administration to the candidate administration site to within 10 minutes after administration, preferably any time from immediately after administration to the candidate administration site to 10 to 120 seconds after administration. When the sense of discomfort occurs, the candidate administration site can be determined as the responsible blood vessel, and therefore, the therapeutic agent of the present invention is administered using the site as the administration site (the above-mentioned site 2).

The present inventor has found that the contrast agent with a higher osmotic pressure than blood and an embolic material with a higher osmotic pressure than blood act on a responsible blood vessel of a sports injury or of a pain due to the sports injury, and cause a sense of discomfort, particularly, a pain similar to the pain (reproduced pain) due to the sports injury in the affected area. The presence or absence of such a reproduced pain can be used as an index to identify a blood vessel responsible for a sports injury or a pain due to the sports injury.

In the present invention, the contrast agent with a higher osmotic pressure than blood and an embolic material with a higher osmotic pressure than blood, which are used to identify the site 2, correspond to a "particulate embolic material administration site determination agent for the therapy of a sports injury or a pain due to the sports injury".

In the present invention, when the presence or absence of an abnormal finding at the candidate administration site is unclear, it is preferable to select whether to administer the particulate embolic material administration site determination agent, based on whether the candidate administration site is a blood vessel that usually feeds the pain site. When the candidate administration site does not coincide with the usual pain site, there may be a disadvantage that excessive embolization to the normal site and a pain or a neurological injury due to the excessive embolization occur, and therefore, it is preferable to select not to use the particulate embolic material administration site determination agent. In this case, the catheter is removed from the candidate administration site, delivered to another candidate administration site, and to confirm an abnormal finding again.

### (Contrast Agents with Higher Osmotic Pressure than Blood)

In the present invention, the term "contrast agent with a higher osmotic pressure than blood" means a contrast agent having an osmotic pressure higher than that of blood in a living body (about 280 mOsm/kg). For example, a contrast agent with a higher osmotic pressure than blood may be 1.2 times or more and 3.0 times or less the osmotic pressure of blood in a living body.

Examples of the contrast agent with a higher osmotic pressure than blood include Omnipaque and Hexabrix.

The contrast agent with a higher osmotic pressure than blood can be mixed with a liquid (physiological saline) that can be administered to a living body. An amount of the contrast agent with a higher osmotic pressure than blood used is not particularly limited as long as the contrast agent can temporarily embolize the candidate administration site or cause a reproduced pain, and can be set according to the blood vessel diameter or the like of the candidate administration site.

For example, the amount of contrast agent with a higher osmotic pressure than blood used (dose to the administration candidate site) may be 0.2 to 3 mL (volume). The contrast agent may be administered either as a single dose or in divided doses.

### (Embolic Material with Higher Osmotic Pressure than Blood)

In the present invention, the term "embolic material with a higher osmotic pressure than blood" means an embolic material having an osmotic pressure higher than that of blood in a living body (about 280 mOsm/kg). Such embolic materials include substances that, when administered to an artery of a person having a healthy body (a body that does not have a sports injury or a pain due to the sports injury), cause a sense of discomfort (a pain, a heat sensation, itching, pressure sensation, etc.) at the site of administration within 10 minutes after administration.

An example of the embolic material with a higher osmotic pressure than blood is Tienam (cilastatin sodium).

The average particle size and shape of the embolic material with a higher osmotic pressure than blood are not particularly limited, and may be the same as those of the embolic material contained in the therapeutic agent of the present invention. The lower limit of the average particle size of the embolic material with a higher osmotic pressure than blood may be, for example, 10 µm or more, 20 µm or more, or 30 µm or more. The upper limit of the average particle size of the embolic material with a higher osmotic pressure than blood may be, for example, 500 µm or less, 200 µm or less, or 100 µm or less.

The embolic material with a higher osmotic pressure than blood can be mixed with a liquid (physiological saline) that can be administered to a living body. An amount of the embolic material with a higher osmotic pressure than blood to be used is not particularly limited as long as the embolic material can temporarily embolize the candidate administration site, and can be set according to the particle size of the embolic material with a higher osmotic pressure than blood, the blood vessel diameter of the candidate administration site, and the like.

For example, the amount of embolic material with a higher osmotic pressure than blood to be used (dose to the administration candidate site) may be 10 to 500 mg (dry weight). The substance may be administered either as a single dose or in divided doses.

### (Dosage, etc. of Therapeutic Agent)

The administration rate and the administration frequency of the therapeutic agent of the present invention can be appropriately set according to the condition of the patient (age, weight, severity of symptoms, etc.).

The end of administration of the therapeutic agent of the present invention is performed by any operation capable of terminating the injection of the therapeutic agent into the administration site. As such an operation, for example, pulling out the outer cylinder from the administration site and performing compression hemostasis can be exemplified.

### <Therapeutic Effect of Therapeutic Agent of Present Invention>

The therapeutic effect of the therapeutic agent of the present invention can be evaluated by any method for evaluating pain. Examples thereof include a method based on "numerical rating scale (NRS) score" and a method in which a patient subjectively answers the degree of pain relief ("Patients Global Impression of Change").

### EXAMPLES

Hereinafter, the present invention will be described with reference to Examples in more detail, but the present invention is not limited to these Examples.

In all of the following tests, a sense of discomfort (pain, heat sensation, etc.) occurred in the affected area and the pain site within 10 to 120 seconds (at least within 10 minutes after administration) from immediately after administration of the particulate embolic material administration site determination agent in most of the patients of "(2-2) Case where Presence or Absence of Abnormal Finding was Unknown".

### <Preparation of Therapeutic Agents>

In this Example, one of the following was used as the embolic material. Each embolic material was prepared as a 1 g/10 mL aqueous solution.

### (1) Imipenem/Cilastatin

"Primaxin" (manufactured by Merck & Co., Inc.) was used as imipenem/cilastatin. This embolic material had an average particle diameter of 70 µm or less as measured by a laser diffraction-type particle size distribution measuring device ("SALD" series, manufactured by Shimadzu Corporation).

### (2) Gelatin Preparation

"IPZA 100-300" and "IPZA 300-500" from Engain, or "Nexsphere" manufactured by Nextbiomedical, which are commercially available gelatin preparations, were used. Both of these satisfy the following (A) and (B).
(A) 0.2 g of the substance was mixed in 30 mL of physiological saline at 37°C, and the "diffraction intensity/scattered light intensity" after 10 minutes was 10% or more of the "diffraction intensity/scattered light intensity" immediately after mixing.
(B) 0.2 g of the substance was mixed in 30 mL of physiological saline at 37°C, and the "diffraction intensity/scattered light intensity" after one hour was 90% or less of the "diffraction intensity/scattered light intensity" immediately after mixing.
(The "diffraction intensity/scattered light intensity" was measured using a laser diffraction particle size distribution analyzer "SALD-2300" (manufactured by Shimadzu Corporation) and a batch cell "BC23" (manufactured by Shimadzu Corporation) at a refractive index of 1.65 to 0.20i.)

In this Example, Iopaque (manufactured by Fuji Pharmaceutical Co., Ltd.) was used as a contrast agent. The contrast agent was used as an admixture with the embolic material. A mixing ratio (mass ratio of active ingredients) was set at 20 mL of the contrast agent with respect to 1 g of the embolic material. The resulting solutions were subjected to the following tests as the therapeutic agent.

### <Preparation of Particulate Embolic Material Administration Site Determination Agent>

In this Example, the following components were prepared as the particulate embolic material administration site determination agents.

### (1) Contrast Agent with Higher Osmotic Pressure than Blood

"Iopark" was used as the contrast agent. This contrast agent has an osmotic pressure about two times higher than that of blood.

### (2) Embolic Material with Higher Osmotic Pressure than Blood

"Tienam" (average particle size: 40 µm) was used as the embolic material with a higher osmotic pressure than blood. This embolic material with a higher osmotic pressure than blood was prepared as a 1 g/10 mL aqueous solution.

### <Test 1: Therapy of Shoulder Sports Injury>

In each patient with a shoulder sports injury or a pain due to the sports injury, the administration site of the therapeutic agent was identified, and therapy with the therapeutic agent of the present invention was performed.

### (1) Patients

Patients (10 people) were those who do sports on a regular basis and with whom abnormal findings were observed in the shoulders. These patients were associated with one of the following diseases: a disabled throwing shoulder, a shoulder impingement syndrome, a painful rotator cuff tear, a SLAP lesion, rotator interval inflammation, biceps long head tendinitis, calcific tendinitis, and rotator cuff tendinitis.

### (2) Identification of Administration Sites of Therapeutic Agents and Therapy

A catheter was inserted from a position where access to the artery was possible (radial artery, inguinal artery, etc.), and advanced to the axillary artery or subclavian artery, and angiography was performed. Arteries (candidate administration sites) that are targeted in the shoulder sports injury are usually a subscapular artery, an acromiothoracic artery, a coracoid branch, a scapular circumflex artery, an anterior circumflex humeral artery, or an aposterior circumflex humeral artery. All of these correspond to "the artery that feeds an affected area of a shoulder sports injury and/or the artery that feeds a pain site due to the shoulder sports injury". Therefore, a catheter was delivered to these arteries and angiography was selectively performed.

### (2-1) Case where Abnormal Finding was Observed

When a clear abnormal finding was observed as a result of angiography, the site was determined as the administration site for the therapeutic agent. Then, the therapeutic agent (about 0.2 mL) was administered to the administration site.

### (2-2) Case where Presence or Absence of Abnormal Finding was Unknown

As a result of angiography, even when the presence or absence of abnormal finding was unknown, when the candidate administration site was considered to have a possibility of feeding the pain site, one of the particulate embolic material administration site determination agents (about 0.2 mL) was administered.

When a sense of discomfort (pain, heat sensation, etc.) occurred in the affected area or the pain site immediately after the administration of the particulate embolic material administration site determination agent to within 10 minutes after the administration, the candidate administration site was determined as a blood vessel (a responsible blood vessel) of a responsible site of the injury or pain, that is, the administration site. Then, the therapeutic agent (about 0.2 mL) was administered to the administration site after a short time (at least 5 minutes or more after the administration) from the administration of the particulate embolic material administration site determination agent. Depending on the degree of injury or pain, and/or depending on the degree of reduction in angiographic abnormalities, the therapeutic agent was administered as a single dose or multiple doses. When multiple doses were administered, the dose interval was set to 5 minutes.

On the other hand, in the case where a sense of discomfort (pain, heat sensation, etc.) did not occur in the affected area or pain site immediately after the administration of the particulate embolic material administration site determination agent to within 10 minutes after the administration, the candidate administration site was determined as a site not involved in the injury or the pain, and no further administration was performed. In this case, the above-described step (2) was repeated at another candidate administration site to identify the administration site, and the therapeutic agent (about 0.2 mL) was administered.

### (2-3) Case where No Abnormal Findings were Observed

In the case where no abnormal findings were observed, the imaging site of the angiography was changed without administering either the particulate embolic material administration site determination agent or the therapeutic agent, the above-described step (2) was repeated to identify the administration site, and then the therapeutic agent (about 0.2 mL) was administered.

### (3) Results

At a time point of two months after the completion of the administration of the therapeutic agent, each patient reported the pain after the treatment as an integer (0 or more and 10 or less) according to a known pain evaluation method (NRS score), by rating the pain before the therapy as "10". As a result, 80% or more of patients answered that symptoms and pains had decreased to half or less. In addition, no significant complications occurred in any of the patients. Regardless of the type of the particulate embolic material administration site determination agent, the therapeutic agent to the administration site identified by the determination agent was effective.

### <Test 2: Therapy of Elbow Sports Injury>

In each patient with an elbow sports injury or a pain due to the sports injury, the administration site of the therapeutic agent was identified, and therapy with the therapeutic agent of the present invention was performed.

### (1) Patients

Patients (45 people) were those who do sports on a regular basis and with whom abnormal findings were observed in the elbows. These patients were associated with one of the following diseases: a medial ulnar collateral ligament injury, olecranon stress fracture, lateral humeral epicondylitis, medial humeral epicondylitis, an infrapatellar synovial fold injury, elbow-joint synovial inflammation, osteochondritis dissecans, and other pains in the elbow joint due to sports.

### (2) Identification of Administration Sites of Therapeutic Agent and Therapy

A catheter was inserted from a position where access to the artery was possible (radial artery, inguinal artery, etc.), and advanced to the brachial artery, and angiography was performed. Arteries (candidate administration sites) that are targeted in the elbow sports injury are usually an arteria profunda brachii, a radial collateral artery, an ulnar collateral artery, a radial recurrent artery, a recurrent interosseous artery, or a recurrent ulnar artery. All of these correspond to "the artery that feeds an affected area of an elbow sports injury and/or the artery that feeds a pain site due to the elbow sports injury". Therefore, a catheter was delivered to these arteries and angiography was selectively performed.

### (2-1) Case where Abnormal Finding was Observed

When a clear abnormal finding was observed as a result of angiography, the site was determined as the administration site for the therapeutic agent. Then, the therapeutic agent (about 0.2 mL) was administered to the administration site.

### (2-2) Case where Presence or Absence of Abnormal Finding was Unknown

As a result of angiography, even when the presence or absence of abnormal finding was unknown, when the candidate administration site was considered to have a possibility of feeding the pain site, one of the particulate embolic material administration site determination agents (about 0.2 mL) was administered.

When a sense of discomfort (pain, heat sensation, etc.) occurred in the affected area or the pain site immediately after the administration of the particulate embolic material administration site determination agent to within 10 minutes after the administration, the candidate administration site was determined as a blood vessel (a responsible blood vessel) of a responsible site of the disorder or pain, that is, the administration site. Then, the therapeutic agent (about 0.2 mL) was administered to the administration site after a short time (at least 5 minutes or more after the administration) from the administration of the particulate embolic material administration site determination agent. Depending on the degree of disorder or pain, and/or depending on the degree of reduction in angiographic abnormalities, the therapeutic agent was administered as a single dose or multiple doses. When multiple doses were administered, the dose interval was set to 5 minutes.

On the other hand, in the case where a sense of discomfort (pain, heat sensation, etc.) did not occur in the affected area or pain site immediately after the administration of the particulate embolic material administration site determination agent to within 10 minutes after the administration, the candidate administration site was determined as a site not involved in the disorder or the pain, and no further administration was performed. In this case, the above-described step (2) was repeated at another candidate administration site to identify the administration site, and the therapeutic agent (about 0.2 mL) was administered.

### (2-3) Case where No Abnormal Findings were Observed

In the case where no abnormal findings were observed, the imaging site of the angiography was changed without administering either the particulate embolic material administration site determination agent or the therapeutic agent, the above-described step (2) was repeated to identify the administration site, and then the therapeutic agent (about 0.2 mL) was administered.

### (3) Results

At a time point of three months after the completion of the administration of the therapeutic agent, each patient reported the pain after the treatment as an integer (0 or more and 10 or less) according to a known pain evaluation method (NRS score), by rating the pain before the therapy as "10". As a result, 80% or more of patients answered that symptoms and pains had decreased to half or less. In addition, no significant complications occurred in any of the patients. Regardless of the type of the particulate embolic material administration site determination agent, the therapeutic agent to the administration site identified by the determination agent was effective.

### <Test 3: Therapy of Lower Back and/or Upper Back Sports Injury>

In each patient with a lower back and/or upper back sports injury or a pain due to the sports injury, the administration site of the therapeutic agent was identified, and therapy with the therapeutic agent of the present invention was performed.

### (1) Patients

Patients (15 people) were those who do sports on a regular basis and with whom abnormal findings were observed in the lower back and/or the upper back. These patients were associated with one of the following diseases: lumbar spondylolysis, intervertebral joint backache, lumbar disc arthritis, thoracic disc arthritis, fascia of muscles backache, interspinal desmitis, lumber disc herniation, lumbar disc endplate inflammation, sacroiliac joint inflammation, and other backaches due to sports.

### (2) Identification of Administration Sites of Therapeutic Agents and Therapy

A catheter was inserted from a position where access to the artery was possible (inguinal artery, etc.), and advanced to the abdominal aorta, and angiography was performed. Arteries (candidate administration sites) that are targeted in the lower back and/or upper back sports injuries are usually left and right first to fourth lumbar arteries, an iliolumbar artery, a middle sacral artery, a lateral sacral artery, a superior gluteal artery, an inferior gluteal artery, and an intercostal artery. All of these correspond to "the artery that feeds an affected area of a lower back and/or upper back sports injury and/or the artery that feeds a pain site due to the lower back and/or upper back sports injury". Therefore, a catheter was delivered to these arteries and angiography was selectively performed.

Note that when the particulate embolic material administration site determination agent or the therapeutic agent was to be administered to the lumbar artery or the intercostal artery, the administration was performed after confirming that an artery (Adamkiewicz artery) to the spinal cord was not present. When the artery to the spinal cord was confirmed to be present, administration was suspended, or the microcatheter was sufficiently advanced to avoid the artery, further advanced to a position where administration was possible, and then administration was performed.

### (2-1) Case where Abnormal Finding was Observed

When a clear abnormal finding was observed as a result of angiography, the site was determined as the administration site for the therapeutic agent. Then, the therapeutic agent (about 0.2 mL to 0.5 mL) was administered to the administration site.

### (2-2) Case where Presence or Absence of Abnormal Finding was Unknown

As a result of angiography, even when the presence or absence of abnormal finding was unknown, when the candidate administration site was considered to have a possibility of feeding the pain site, one of the particulate embolic material administration site determination agents (about 0.2 mL to 0.5 mL) was administered.

When a sense of discomfort (pain, heat sensation, etc.) occurred in the affected area or the pain site immediately after the administration of the particulate embolic material administration site determination agent to within 10 minutes after the administration, the candidate administration site was determined as a blood vessel (a responsible blood vessel) of a responsible site of the disorder or pain, that is, the administration site. Then, the therapeutic agent (about 0.2 mL to 0.5 mL) was administered to the administration site after a short time (at least 5 minutes or more after the administration) from the administration of the particulate embolic material administration site determination agent. Depending on the degree of disorder or pain, and/or depending on the degree of reduction in angiographic abnormalities, the therapeutic agent was administered as a single dose or multiple doses. When multiple doses were administered, the dose interval was set to 5 minutes.

On the other hand, in the case where a sense of discomfort (pain, heat sensation, etc.) did not occur in the affected area or pain site immediately after the administration of the particulate embolic material administration site determination agent to within 10 minutes after the administration, the candidate administration site was determined as a site not involved in the disorder or the pain, and no further administration was performed. In this case, the above-described step (2) was repeated at another candidate administration site to identify the administration site, and the therapeutic agent (about 0.2 mL to 0.5 mL) was administered.

### (2-3) Case where No Abnormal Findings were Observed

In the case where no abnormal findings were observed, the imaging site of the angiography was changed without administering either the particulate embolic material administration site determination agent or the therapeutic agent, the above-described step (2) was repeated to identify the administration site, and then the therapeutic agent (about 0.2 mL to 0.5 mL) was administered.

### (3) Results

At a time point of three months after the completion of the administration of the therapeutic agent, each patient reported the pain after the treatment as an integer (0 or more and 10 or less) according to a known pain evaluation method (NRS score), by rating the pain before the therapy as "10". As a result, 80% or more of patients answered that symptoms and pains had decreased to half or less. In addition, no significant complications occurred in any of the patients. Regardless of the type of the particulate embolic material administration site determination agent, the therapeutic agent to the administration site identified by the determination agent was effective.

### <Test 4: Therapy of Hip Joint Sports Injury>

In each patient with a hip joint sports injury or a pain due to the sports injury, the administration site of the therapeutic agent was identified, and therapy with the therapeutic agent of the present invention was performed.

### (1) Patients

Patients (17 people) were those who do sports on a regular basis and with whom abnormal findings were observed in the hip joints. These patients were associated with one of the following diseases: hamstrings enthesitis, hip joint synovitis, a hip joint lip lesion, a hip joint impingement syndrome, a groin pain syndrome, great trochanter bursitis, and other pains of the hip joint due to sports.

### (2) Identification of Administration Sites of Therapeutic Agent and Therapy

A catheter was inserted from a position where access to the artery was possible (inguinal artery, etc.), and advanced to an internal iliac artery, and angiography was performed. Arteries (candidate administration sites) that are targeted in the hip joint sports injury are usually a superior gluteal artery, an inferior gluteal artery, an obturator artery, a deep iliac circumflex artery, an inferior epigastric artery, a medial circumflex femoral artery, and a lateral circumflex femoral artery. All of these correspond to "the artery that feeds an affected area of a hip joint sports injury and/or the artery that feeds a pain site due to the hip joint sports injury". Therefore, a catheter was delivered to these arteries and angiography was selectively performed.

### (2-1) Case where Abnormal Finding was Observed

When a clear abnormal finding was observed as a result of angiography, the site was determined as the administration site for the therapeutic agent. Then, the therapeutic agent (about 0.2 mL) was administered to the administration site.

### (2-2) Case where Presence or Absence of Abnormal Finding was Unknown

As a result of angiography, even when the presence or absence of abnormal finding was unknown, when the candidate administration site was considered to have a possibility of feeding the pain site, one of the particulate embolic material administration site determination agents (about 0.2 mL) was administered.

When a sense of discomfort (pain, heat sensation, etc.) occurred in the affected area or the pain site immediately after the administration of the particulate embolic material administration site determination agent to within 10 minutes after the administration, the candidate administration site was determined as a blood vessel (a responsible blood vessel) of a responsible site of the disorder or pain, that is, the administration site. Then, the therapeutic agent (about 0.2 mL) was administered to the administration site after a short time (at least 5 minutes or more after the administration) from the administration of the particulate embolic material administration site determination agent. Depending on the degree of disorder or pain, and/or depending on the degree of reduction in angiographic abnormalities, the therapeutic agent was administered as a single dose or multiple doses. When multiple doses were administered, the dose interval was set to 5 minutes.

On the other hand, in the case where a sense of discomfort (pain, heat sensation, etc.) did not occur in the affected area or pain site immediately after the administration of the particulate embolic material administration site determination agent to within 10 minutes after the administration, the candidate administration site was determined as a site not involved in the disorder or the pain, and no further administration was performed. In this case, the above-described step (2) was repeated at another candidate administration site to identify the administration site, and the therapeutic agent (about 0.2 mL) was administered.

### (2-3) Case where No Abnormal Findings were Observed

In the case where no abnormal findings were observed, the imaging site of the angiography was changed without administering either the particulate embolic material administration site determination agent or the therapeutic agent, the above-described step (2) was repeated to identify the administration site, and then the therapeutic agent (about 0.2 mL) was administered.

### (3) Results

At a time point of three months after the completion of the administration of the therapeutic agent, each patient reported the pain after the treatment as an integer (0 or more and 10 or less) according to a known pain evaluation method (NRS score), by rating the pain before the therapy as "10". As a result, 80% or more of patients answered that symptoms and pains had decreased to half or less. In addition, no significant complications occurred in any of the patients. Regardless of the type of the particulate embolic material administration site determination agent, the therapeutic agent to the administration site identified by the determination agent was effective.

### <Test 5: Therapy of Knee Joint Sports Injury>

In each patient with a knee joint sports injury or a pain due to the sports injury, the administration site of the therapeutic agent was identified, and therapy with the therapeutic agent of the present invention was performed.

### (1) Patients

Patients (49 people) were those who do sports on a regular basis and with whom abnormal findings were observed in the knee joints. These patients were associated with one of the following diseases: patellar tendinitis, patellofemoral desmitis, Iliotibial desmitis, pes anserinus bursitis, subpatellar panniculitis, a medial collateral ligament lesion, a semi-lunar disc lesion, Osgood disease, a synovial membrane disorder, patella partita, and other pains in the knee joint due to sports.

### (2) Identification of Administration Sites of Therapeutic Agent and Therapy

A catheter was inserted from a position where access to the artery was possible (inguinal artery, etc.), and advanced to the popliteal artery, and angiography was performed. Arteries (candidate administration sites) that are targeted in the knee joint sports injury are usually a descending artery of knee, an upper medial artery of knee, a superolateral artery of knee, a median artery of knee, a lower lateral artery of knee, an inferior medial artery of knee, an anterior tibial recurrent artery, and a suprapatellar artery. All of these correspond to "the artery that feeds an affected area of a knee joint sports injury and/or the artery that feeds a pain site due to a knee joint sports injury". Therefore, a catheter was delivered to these arteries and angiography was selectively performed.

### (2-1) Case where Abnormal Finding was Observed

When a clear abnormal finding was observed as a result of angiography, the site was determined as the administration site for the therapeutic agent. Then, the therapeutic agent (about 0.2 mL) was administered to the administration site.

### (2-2) Case where Presence or Absence of Abnormal Finding was Unknown

As a result of angiography, even when the presence or absence of abnormal finding was unknown, when the candidate administration site was considered to have a possibility of feeding the pain site, one of the particulate embolic material administration site determination agents (about 0.2 mL) was administered.

When a sense of discomfort (pain, heat sensation, etc.) occurred in the affected area or the pain site immediately after the administration of the particulate embolic material administration site determination agent to within 10 minutes after the administration, the candidate administration site was determined as a blood vessel (a responsible blood vessel) of a responsible site of the disorder or pain, that is, the administration site. Then, the therapeutic agent (about 0.2 mL) was administered to the administration site after a short time (at least 5 minutes or more after the administration) from the administration of the particulate embolic material administration site determination agent. Depending on the degree of disorder or pain, and/or depending on the degree of reduction in angiographic abnormalities, the therapeutic agent was administered as a single dose or multiple doses. When multiple doses were administered, the dose interval was set to 5 minutes.

On the other hand, in the case where a sense of discomfort (pain, heat sensation, etc.) did not occur in the affected area or pain site immediately after the administration of the particulate embolic material administration site determination agent to within 10 minutes after the administration, the candidate administration site was determined as a site not involved in the disorder or the pain, and no further administration was performed. In this case, the above-described step (2) was repeated at another candidate administration site to identify the administration site, and the therapeutic agent (about 0.2 mL) was administered.

### (2-3) Case where No Abnormal Findings were Observed

In the case where no abnormal findings were observed, the imaging site of the angiography was changed without administering either the particulate embolic material administration site determination agent or the therapeutic agent, the above-described step (2) was repeated to identify the administration site, and then the therapeutic agent (about 0.2 mL) was administered.

### (3) Results

At a time point of three months after the completion of the administration of the therapeutic agent, each patient reported the pain after the treatment as an integer (0 or more and 10 or less) according to a known pain evaluation method (NRS score), by rating the pain before the therapy as "10".

As a result, 80% or more of patients answered that symptoms and pains had decreased to half or less. In addition, no significant complications occurred in any of the patients. Regardless of the type of the particulate embolic material administration site determination agent, the therapeutic agent to the administration site identified by the determination agent was effective.

### <Test 6: Therapy of Foot Joint and/or Foot Sports Injury>

In each patient with a foot joint and/or foot sports injury or a pain due to the sports injury, the administration site of the therapeutic agent was identified, and therapy with the therapeutic agent of the present invention was performed.

### (1) Patients

Patients (56 people) were those who do sports on a regular basis and with whom abnormal findings were observed in the foot joint and/or foot. These patients were associated with one of the following diseases: Achilles tendinitis, plantar aponeurosis, painful os tibiale externum, foot joint impingement syndrome, Hallux valgus, Morton's neuroma, tibialis posterior tendinitis, foot joint synovitis, MP arthritis, and other pains in the foot joint or foot due to sports.

### (2) Identification of Administration Sites of Therapeutic Agents and Therapy

A catheter was inserted from a position where access to the artery was possible (inguinal artery, etc.), and advanced to the popliteal artery, and angiography was performed. Arteries (candidate administration sites) that are targeted in the foot joint and/or foot sports injury are usually an anterior tibial artery, a posterior tibial artery, and a peroneal artery. All of these correspond to "the artery that feeds an affected area of a foot joint and/or foot sports injury and/or the artery that feeds a pain site due to a foot joint and/or foot sports injury". Therefore, a catheter was delivered to these arteries and angiography was selectively performed.

### (2-1) Case where Abnormal Finding was Observed

When a clear abnormal finding was observed as a result of angiography, the site was determined as the administration site for the therapeutic agent. Then, the therapeutic agent (about 0.2 mL) was administered to the administration site.

### (2-2) Case where Presence or Absence of Abnormal Finding was Unknown

As a result of angiography, even when the presence or absence of abnormal finding was unknown, when the candidate administration site was considered to have a possibility of feeding the pain site, one of the particulate embolic material administration site determination agents (about 0.2 mL) was administered.

When a sense of discomfort (pain, heat sensation, etc.) occurred in the affected area or the pain site immediately after the administration of the particulate embolic material administration site determination agent to within 10 minutes after the administration, the candidate administration site was determined as a blood vessel (a responsible blood vessel) of a responsible site of the disorder or pain, that is, the administration site. Then, the therapeutic agent (about 0.2 mL) was administered to the administration site after a short time (at least 5 minutes or more after the administration) from the administration of the particulate embolic material administration site determination agent. Depending on the degree of disorder or pain, and/or depending on the degree of reduction in angiographic abnormalities, the therapeutic agent was administered as a single dose or multiple doses. When multiple doses were administered, the dose interval was set to 5 minutes.

On the other hand, in the case where a sense of discomfort (pain, heat sensation, etc.) did not occur in the affected area or pain site immediately after the administration of the particulate embolic material administration site determination agent to within 10 minutes after the administration, the candidate administration site was determined as a site not involved in the disorder or the pain, and no further administration was performed. In this case, the above-described step (2) was repeated at another candidate administration site to identify the administration site, and the therapeutic agent (about 0.2 mL) was administered.

### (2-3) Case where No Abnormal Findings were Observed

In the case where no abnormal findings were observed, the imaging site of the angiography was changed without administering either the particulate embolic material administration site determination agent or the therapeutic agent, the above-described step (2) was repeated to identify the administration site, and then the therapeutic agent (about 0.2 mL) was administered.

### (3) Results

At a time point of three months after the completion of the administration of the therapeutic agent, each patient reported the pain after the treatment as an integer (0 or more and 10 or less) according to a known pain evaluation method (NRS score), by rating the pain before the therapy as "10". As a result, 80% or more of patients answered that symptoms and pains had decreased to half or less. In addition, no significant complications occurred in any of the patients. Regardless of the type of the particulate embolic material administration site determination agent, the therapeutic agent to the administration site identified by the determination agent was effective.

### <Test 7: Therapy of Hand joint Sports Injury>

In each patient with a hand joint sports injury or a pain due to the sports injury, the administration site of the therapeutic agent was identified, and therapy with the therapeutic agent of the present invention was performed.

### (1) Patients

Patients (20 people) were those who do sports on a regular basis and with whom abnormal findings were observed in the hand joints. These patients were associated with one of the following diseases: a TFCC lesion, tendon sheath inflammation, navicular bone fracture, hand joint synovitis, and other pains in the hand joint due to sports.

### (2) Identification of Administration Sites of Therapeutic Agents and Therapy

A catheter was inserted from a position where access to the artery was possible (radial artery, inguinal artery, etc.), and advanced to the popliteal artery, and angiography was performed. Arteries (candidate administration sites) that are targeted in the hand joint sports injury are usually a radial artery, an ulnar artery, and an interosseous artery. All of these correspond to "the artery that feeds an affected area of a hand joint sports injury and/or the artery that feeds a pain site due to a hand joint sports injury". Therefore, a catheter was delivered to these arteries and angiography was selectively performed.

### (2-1) Case where Abnormal Finding was Observed

When a clear abnormal finding was observed as a result of angiography, the site was determined as the administration site for the therapeutic agent. Then, the therapeutic agent (about 0.2 mL) was administered to the administration site.

### (2-2) Case where Presence or Absence of Abnormal Finding was Unknown

As a result of angiography, even when the presence or absence of abnormal finding was unknown, when the candidate administration site was considered to have a possibility of feeding the pain site, one of the particulate embolic material administration site determination agents (about 0.2 mL) was administered.

When a sense of discomfort (pain, heat sensation, etc.) occurred in the affected area or the pain site immediately after the administration of the particulate embolic material administration site determination agent to within 10 minutes after the administration, the candidate administration site was determined as a blood vessel (a responsible blood vessel) of a responsible site of the disorder or pain, that is, the administration site. Then, the therapeutic agent (about 0.2 mL) was administered to the administration site after a short time (at least 5 minutes or more after the administration) from the administration of the particulate embolic material administration site determination agent. Depending on the degree of disorder or pain, and/or depending on the degree of reduction in angiographic abnormalities, the therapeutic agent was administered as a single dose or multiple doses. When multiple doses were administered, the dose interval was set to 5 minutes.

On the other hand, in the case where a sense of discomfort (pain, heat sensation, etc.) did not occur in the affected area or pain site immediately after the administration of the particulate embolic material administration site determination agent to within 10 minutes after the administration, the candidate administration site was determined as a site not involved in the disorder or the pain, and no further administration was performed. In this case, the above-described step (2) was repeated at another candidate administration site to identify the administration site, and the therapeutic agent (about 0.2 mL) was administered.

### (2-3) Case where No Abnormal Findings were Observed

In the case where no abnormal findings were observed, the imaging site of the angiography was changed without administering either the particulate embolic material administration site determination agent or the therapeutic agent, the above-described step (2) was repeated to identify the administration site, and then the therapeutic agent (about 0.2 mL) was administered.

### (3) Results

At a time point of three months after the completion of the administration of the therapeutic agent, each patient reported the pain after the treatment as an integer (0 or more and 10 or less) according to a known pain evaluation method (NRS score), by rating the pain before the therapy as "10". As a result, 80% or more of patients answered that symptoms and pain had decreased to half or less. In addition, no significant complications occurred in any of the patients. Regardless of the type of the particulate embolic material administration site determination agent, the therapeutic agent to the administration site identified by the determination agent was effective.

### <Test 8: Therapy of Shoulder Sports Injury>

In each patient with a repetitive muscular detachment, fatigue fracture, fracture healing delay, a false joint, and the like, the administration site of the therapeutic agent was identified, and therapy with the therapeutic agent of the present invention was performed. Note that these symptoms are included in sports injuries, but are not always accompanied by a pain.

### (1) Patients

Patients (12 people) were those who had a repetitive muscular detachment, fatigue fracture, fracture healing delay, or a pseudo-joint in arbitrary sites.

### (2) Identification of Administration Sites of Therapeutic Agent and Therapy

A catheter was inserted from a position where access to the artery was possible (inguinal artery, radial artery, etc.), and advanced to the popliteal artery, and angiography was performed. Arteries (candidate administration sites) that are targeted in the repetitive muscular detachment, fatigue fracture, fracture healing delay, or pseudo-joint vary depending on the site, and thus the artery (candidate administration site) considered to feed the affected area was selected, the catheter was delivered, and the angiography was selectively performed.

### (2-1) Case where Abnormal Finding was Observed

When a clear abnormal finding was observed as a result of angiography, the site was determined as the administration site for the therapeutic agent. Then, the therapeutic agent (about 0.2 mL) was administered to the administration site.

### (2-2) Case where Presence or Absence of Abnormal Finding was Unknown

As a result of angiography, even when the presence or absence of abnormal finding was unknown, when the candidate administration site was considered to have a possibility of feeding the pain site, one of the particulate embolic material administration site determination agents (about 0.2 mL) was administered.

When a sense of discomfort (pain, heat sensation, etc.) occurred in the affected area or the pain site immediately after the administration of the particulate embolic material administration site determination agent to within 10 minutes after the administration, the candidate administration site was determined as a blood vessel (a responsible blood vessel) of a responsible site of the disorder or pain, that is, the administration site. Then, the therapeutic agent (about 0.2 mL) was administered to the administration site after a short time (at least 5 minutes or more after the administration) from the administration of the particulate embolic material administration site determination agent. Depending on the degree of disorder or pain, and/or depending on the degree of reduction in angiographic abnormalities, the therapeutic agent was administered as a single dose or multiple doses. When multiple doses were administered, the dose interval was set to 5 minutes.

On the other hand, in the case where a sense of discomfort (pain, heat sensation, etc.) did not occur in the affected area or pain site immediately after the administration of the particulate embolic material administration site determination agent to within 10 minutes after the administration, the candidate administration site was determined as a site not involved in the disorder or the pain, and no further administration was performed. In this case, the above-described step (2) was repeated at another candidate administration site to identify the administration site, and the therapeutic agent (about 0.2 mL) was administered.

### (2-3) Case where No Abnormal Findings were Observed

In the case where no abnormal findings were observed, the imaging site of the angiography was changed without administering either the particulate embolic material administration site determination agent or the therapeutic agent, the above-described step (2) was repeated to identify the administration site, and then the therapeutic agent (about 0.2 mL) was administered.

### (3) Results

At a time point of three months after the completion of the administration of the therapeutic agent, each patient reported the pain after the treatment as an integer (0 or more and 10 or less) according to a known pain evaluation method (NRS score), by rating the pain before the therapy as "10". As a result, 80% or more of patients answered that symptoms and pain had decreased to half or less. In addition, no significant complications occurred in any of the patients. Regardless of the type of the particulate embolic material administration site determination agent, the therapeutic agent to the administration site identified by the determination agent was effective.

### <Reference Test: Confirmation of Effect by Use of Particulate Embolic Material Administration Site Determination Agent>

In the same test as described above, when the presence or absence of abnormal findings based on angiography was unknown (corresponding to "(2-2) Case where Presence or Absence of Abnormal Finding was Unknown"), the therapeutic agent was directly administered to the candidate administration site without administering the particulate embolic substance administration site determination agent. As a result, the evaluation result by the NRS score was low, and only about 50% of patients answered that symptoms and pains had decreased to half or less.

## Claims

1. A therapeutic agent for a sports injury or a pain due to the sports injury,
the therapeutic agent comprising a particulate embolic material having an average particle size of 10 µm or more and 500 µm or less,
wherein the therapeutic agent is administered into an artery that feeds an affected area of the sports injury and/or in an artery that feeds a pain site due to the sports injury.

2. A therapeutic agent for a sports injury or a pain due to the sports injury,
comprising a particulate embolic material having an average particle size of 10 µm or more and 500 µm or less,
wherein the therapeutic agent is administered to one or more of the following sites:
(1) a site for which an abnormal finding is recognized based on imaging of blood vessels at a candidate administration site located in an artery that feeds an affected area of the sports injury and/or in an artery that feeds a pain site due to the sports injury or
(2) a site where a sense of discomfort occurs in an affected area after a contrast agent with a higher osmotic pressure than blood or an embolic material with a higher osmotic pressure than blood is administered to the candidate administration site.

3. A particulate embolic material administration site determination agent for treating a sports injury or a pain due to the sports injury,
the agent comprising a contrast agent with a higher osmotic pressure than blood or an embolic material with a higher osmotic pressure than blood,
wherein the agent is used for determining a candidate administration site of the particulate embolic material based on whether or not the agent administered to the candidate administration site causes a sense of discomfort in an affected area,
the candidate administration site is located in an artery that feeds the affected area of the sports injury and/or in an artery that feeds a pain site due to the sports injury,
the particulate embolic material has an average particle diameter of 10 µm or more and 500 µm or less.
